Europäisches Patentamt

European Patent Office

Office européen des brevets

(11) Publication number: **0 280 807**
**A1**

# EUROPEAN PATENT APPLICATION

(21) Application number: 87308009.7

(22) Date of filing: 10.09.87

(51) Int. Cl.⁴: **A61K 7/48** , A61K 47/00 , A61K 9/06 , C08L 5/08 , //(C08L5/08,71:00)

The title of the invention has been amended (Guidelines for Examination in the EPO, A-III, 7.3).

(30) Priority: **11.12.86 US 940624**

(43) Date of publication of application:
07.09.88 Bulletin 88/36

(84) Designated Contracting States:
BE CH DE FR GB IT LI NL SE

(71) Applicant: **Biomatrix, Inc.**
**65 Railroad Avenue**
**Ridgefield N.J. 07657(US)**

(72) Inventor: **Balazs, Endre A.**
**200 Old Palisades Road**
**Ft. Lee New Jersey 07024(US)**
Inventor: **Leshchiner, Adolf**
**623 Prospect Avenue**
**Fairview New Jersey 07022(US)**

(74) Representative: **Allam, Peter Clerk et al**
**LLOYD WISE, TREGEAR & CO. Norman**
**House 105-109 Strand**
**London WC2R 0AE(GB)**

(54) Hyaluronate or hylan poly(ethylene oxide) mixtures.

(57) Disclosed are water-based, viscoelastic compositions comprising a mixture of hyaluronic acid or hylan, or salts thereof, and water soluble poly-(ethylene oxides). Also disclosed are cosmetic formulations incorporating said compositions.

EP 0 280 807 A1

# IMPROVED HYALURONATE OR HYLAN-POLY(ETHYLENE OXIDE) MIXTURES

The present invention relates to hyaluronate and hylan-based compositions, and more particularly, to such compositions, including, in addition to hyaluronate or hylan, poly(ethylene oxide).

Hyaluronic acid (hereinafter referred to as "HA") as well as its salts, such as sodium hyaluronate and other alkali metal and alkaline earth metal salts (hereinafter referred to as "NaHA") is a known, naturally occurring high viscosity glycosaminoglycan having alternating $\beta$ 1-3 glucuronidic and $\beta$ 1-4 glucosaminidic bonds. The molecular weight of this material is generally within the range of 50,000 to 8,000,000 (although there are reports of HA having molecular weights as high as 13,000,000) depending on the source, method of isolation and method of determination. It is found in animal tissue, e.g. in umbilical cord, vitreous human, synovial fluid, rooster combs, group A and C hemolytic streptococci and in skin.

The isolation and characterization of HA is described in Meyer et al, J. Biol. Chem. 107, 629 (1934); J. Biol. Chem. 114, 689 (1936); Balazs Fed. Proc. 17, 1086 (1958); Laurent et al; Biochim. Biophys. Acta 42, 476 (1960). The structure of HA was elucidated by Weissmann et al, J. Am. Chem. Soc. 76, 1753 (1954) and Meyer, Fed. Proc. 17, 1075 (1958).

For certain uses, extremely pure HA preparations are required; see, for example Balazs U.S. Patent No. 4,141,973, which describes the preparation and use of such an HA.

Poly(ethylene oxides) are known water soluble non-ionic homopolymers of ethylene oxide having molecular weights up to 5,000,000. Aqueous solutions of these polymers are highly viscoelastic and are known to be used in many areas including adhesives, lubricating agents, coatings and cosmetics.

Poly(ethylene oxide) is also known to form strong association complexes with a large number of other materials, such as urea, phenolics, poly(acrylic acid), etc. The reaction products formed between poly(ethylene oxide) and polymeric polycarboxylic acids are insoluble in hot and cold water (U.S. Patent No. 3,387,061).

Water based, highly viscoelastic substances are very useful in cosmetic formulations because they impart to such formulations a smooth, silky texture which is desirable both in terms of the function and aesthetic appeal of cosmetics. One such substance is Hyaderm® (Biomatrix Inc.) which is described in U.S. Patent No. 4,303,676. These hyaluronate compositions are expensive and thus, the art has been seeking ways to easily provide such substances at relatively low cost.

While U.S. Patent No. 3,387,061 suggests that the reaction products of a poly(ethylene oxide) and polymeric polycarboxylic acids are water insoluble, we previously found, quite unexpectedly, and as described in GB-A-2160097, that when aqueous solutions of a hyaluronic acid salt and a high molecular weight poly(ethylene oxide) are mixed together, no water insoluble products are formed, even though hyaluronic acid is a polymeric polycarboxylic acid. Instead, what is observed is a highly viscoelastic substance in which the viscosity of the mixture is substantially in excess of the sum of the individual viscosities of the hyaluronate and the poly(ethylene oxide). This wholly unexpected property makes the resulting mixtures particularly well suited for use in cosmetic formulations both because of the desirability of the vastly increased viscosity and the reduced cost of the substance as compared with a like amount of hyaluronate composition without the poly(ethylene oxide).

It has now been found, in accordance with the present invention in one of its aspects, that the degree of interaction between the two polymers (HA and poly(ethylene oxide)) does not really depend upon the molecular weight of the hyaluronate which is used. Thus, in GB-A-2160097, the lower limit of the molecular weight of the poly(ethylene oxide) was set at about $1 \times 10^6$ for the reason that a poly(ethylene oxide) of lower molecular weight tended to form unusable precipitates when mixed with the sodium hyaluronates used therein, i.e. those with molecular weights of about $1\text{-}5 \times 10^6$. However, we have now found that compositions having the desired properties can be obtained over a much wider range of molcular weights. Thus, in accordance with one aspect of this invention the HA can have a molecular weight as low as $5 \times 10^4$ and ranging up to as high as $1 \times 10^6$ whilst the molecular weight of the poly(ethylene oxide) can range from $1\text{-}5 \times 10^6$ as in GB-A-216097 or can be of lower molecular weight, down to $1 \times 10^5$.

In addition, it has also been discovered in accordance with a further aspect of this invention that compositions having the desired properties can also be obtained using hylan instead of a hyaluronate. Hylan is a chemically modified substance obtained from a hyaluronate and is described in detail in GB-A-2172295.

Moreover, as described in our co-pending EP-A-0224987, the term "hyaluronan" is increasingly being used in the literature in place of the traditional hyaluronic acid and may be used interchangeably therewith.

Accordingly, the present invention provides, in one aspect thereof, water-based viscoelastic compositions comprising a mixture of a hyaluronate (i.e. a hyaluronan), preferably an alkali metal or alkaline earth metal hyaluronate, and most preferably sodium hyaluronate, having a molecular weight of about $5 \times 10^4$ to about $1 \times 10^6$; with a poly(ethylene oxide) having a molecular weight of about $1 \times 10^5$ to about $5 \times 10^6$.

In a further aspect, the present invention provides water-based, viscoelastic compositions comprising a mixture of a hylan, preferably an alkali metal or alkaline earth metal hylan, and most preferably sodium hylan, with a linear homopolymer of ethylene oxide.

In yet another aspect, the invention provides cosmetic formulations comprising the above-defined compositions in admixture with other, conventional additives used in cosmetics, including for example surfactants, thickeners, moisturizing agents, fibers, pigments and dyes and fragrances.

## DETAILED DESCRIPTION OF THE INVENTION

As noted above, the invention provides in one aspect highly viscoelastic compositions comprising a mixture of a hyaluronate and a poly(ethylene oxide). Generally speaking, hyaluronates are more viscous than poly(ethylene oxides) and poly(ethylene oxides) are more elastic than hyaluronates. Moreover, suitable hyaluronates are quite expensive to produce. Notwithstanding the teaching in prior art U.S. Patent No. 3,387,061, we have found that when a composition according to the invention is produced, the result is a water soluble product having the desirable properties of both the hyaluronate and the poly(ethylene oxide) at a much reduced cost.

As in the original invention described and claimed in GB-A-2160097, as the hyaluronic acid salt there can be used a pure HA salt, for example, an alkali or alkaline earth metal salt; or a mixture of hyaluronates containing proteins and other naturally occurring substances, such as the product described in U.S. Patent No. 4,303,676, the contents of which are incorporated herein by reference. However, in this invention, the hyaluronate used has a molecular weight of about 50,000 up to about 1,000,000.

The poly(ethylene oxide) for use with the hyaluronate can be any water soluble linear homopolymer of ethylene oxide having a molecular weight of about $1 \times 10^5$ -$5 \times 10^6$. Such poly(ethylene oxides) are available from Union Carbide Corporation (Polyox® brand) and a particularly suitable grade of Polyox® is that which is identified as "Polyox® Coagulant" in the Union Carbide Corporation publication entitled "Polyox® -Water Soluble Resins are Unique" c 1967, 1972, 1973, 1978, 1981 by Union Carbide Corporation.

The hylan for use herein may be prepared as described in GB-A-2172295. Stated briefly, hylan is prepared by subjecting HA to a cross-linking reaction in situ, that is, in the animal tissue from which it is obtained before its extraction from such tissue. It is soluble notwithstanding its cross-linked nature because the degree of cross-linking is relatively low as compared with more traditional cross-linked HA. There are no critical limits on the molecular weight of the hylan that may be used herein, but in general it is preferred to use a hylan that has a molecular weight ranging from about $5 \times 10^5$ to about $6 \times 10^6$.

Likewise, there are no critical limits on the molecular weight of the poly(ethylene oxide) that is used in admixture with the hylan, but in general it is preferred to use poly(ethylene oxide) having a molecular weight ranging from about $1 \times 10^5$ -$5 \times 10^6$.

The relative concentration of the components in the mixture i.e. hyaluronate or hylan and poly(ethylene oxide) preferably ranges from 0.01 to 2%. The weight ratio of hyaluronate or hylan to poly(ethylene oxide) in the mixture preferably ranges from 1:50 to 100:1.

The viscoelastic compositions of this invention are useful in the preparation of cosmetic formulations such as, for example, shaving gels, moisturizing creams, moisturizing hand lotions, emollient moisturizing creams and eye drop formulations.

Cosmetic formulations according to the invention can be made containing widely varying amounts of the aforesaid mixtures, e.g. from 0.1 up to about 50% of a hyaluronate or hylan-poly(ethylene oxide) mixture depending on the nature of the cosmetic product and the performance characterisitics desired by such formulation. Again, depending upon the type of formulation, it will contain various cosmetic ingredients such as emollients, sugar alcohols, neutral or anionic polysaccharides, preservatives (which do not react with or degrade the hyaluronate or hylan), fragrances, water and the like. These latter materials are all conventional in the art and well known to cosmetic chemists. Included among the cosmetic formulations are moisturizing creams and lotions, shampoos, liquid soaps, shaving cream, eye cream, lip protective creams and make-up, as well as eye drops.

Thus, the invention also includes within its scope eye drop formulations, including both eye drop formulations containing one or more preservatives to ensure stability of the formulation in an already opened container of the product as well as eye drop formulations in single dose forms without preservative. Such non-preservative-containing formulations are desirable because certain users have eyes that are sensitive to the commonly used preservatives. Such formulatinos according to the invention are feasible because as a result of the very low protein content of the hyaluronate used, the formulation will not support the growth of most microorganisms and, therefore, the need for preservatives is avoided.

For specific examples of cosmetic formulations in which the viscoelastic compositions of this invention may be incorporated, the reader is directed to the Examples in GB-A-2160097.

EXAMPLES

The invention will now be described in greater detail in the following examples which are illustrative of the invention without, however, being a limitation thereof.

In the following examples (Examples 1-14), the data for which appears in Table I, three different samples of sodium hyaluronate were used. The molecular weight of the samples were about $5 \times 10^4$, $2.5 \times 10^5$ and $8 \times 10^5$. These samples were obtained by a conventional hydrolysis of high molecular weight sodium hyaluronate recovered from rooster combs. Three different samples of Polyox® were also used in these examples. The molecular weight of these samples were $5 \times 10^6$, $9 \times 10^5$ and $1 \times 10^5$. Rheological measurements were done using a Bohlin Rheometer at 25°C. The following parameters were measured shear viscosity at shear rate ($\gamma$) 1,95$^1$, dynamic viscosity at frequency of 5 Hz and dynamic storage modulus at frequency of 5 Hz.

In Examples 15-20, a hylan of molecular weight of about $8 \times 10^5$ was used in place of sodium hyaluronate.

TABLE I

RHEOLOGICAL PROPERTIES OF SODIUM HYALURONATE OR HYLAN-POLY(ETHYLENE OXIDE) MIXTURES

| Example # | Mixture composition, mg/ml | | Rheological properties | | | | | |
|---|---|---|---|---|---|---|---|---|
| | Sodium hyaluronate MW/concentration or hylan mw/concentration(*) | Poly(ethylene oxide) MW/concentration | Shear viscosity at 1.9 s$^{-1}$, cPs | | Dynamic viscosity at 5 Hz, cPs | | Storage modulus at 5 Hz, Pa | |
| | | | found | additive | found | additive | found | additive |
| 1 | 50,000 / 5 | — | 5.04 | — | 1.28 | — | 0.094 | — |
| 2 | — | 5,000,000 / 5 | 401.00 | — | 18.10 | — | 1.160 | — |
| 3 | 50,000 / 5 | 5,000,000 / 5 | 653.00 | 406.04 | 52.50 | 19.38 | 1.790 | 1.254 |
| 4 | — | 900,000 / 10 | 33.30 | — | 18.30 | — | 0.324 | — |
| 5 | 50,000 / 5 | 900,000 / 10 | 309.00 | 38.34 | 52.70 | 19.58 | 0.595 | 0.418 |
| 6 | — | 100,000 / 15 | 8.87 | — | 1.36 | — | 0.134 | — |
| 7 | 50,000 / 5 | 100,000 / 15 | 23.00 | 13.91 | 2.87 | 2.64 | 0.221 | 0.228 |
| 8 | — | 5,000,000 / 8 | 991.00 | — | 96.10 | — | 4.600 | — |
| 9 | 250,000 / 2 | — | 21.90 | — | 1.02 | — | 0.139 | — |
| 10 | 250,000 / 2 | 5,000,000 / 8 | 1,260.00 | 1012.90 | 115.00 | 97.12 | 4.430 | 4.739 |
| 11 | 250,000 / 5 | — | 31.40 | — | 3.78 | — | 0.211 | — |
| 12 | 250,000 / 5 | 5,000,000 / 5 | 690.00 | 431.40 | 76.00 | 21.88 | 2.490 | 1.371 |
| 13 | 250,000 / 5 | 900,000 / 10 | 231.00 | 64.70 | 71.70 | 22.08 | 0.657 | 0.534 |
| 14 | 250,000 / 5 | 100,000 / 15 | 27.20 | 40.27 | 10.70 | 5.14 | 0.342 | 0.806 |
| 15 | 800,000 / 5 | — | 180.00 | — | 72.40 | — | 1.540 | — |
| 16 | 800,000 / 5 | 5,000,000 / 5 | 2,810.00 | 581.00 | 272.00 | 90.50 | 12.100 | 2.700 |
| 17 | 800,000 / 5 | 900,000 / 10 | 1,240.00 | 213.30 | 303.00 | 90.70 | 7.420 | 1.864 |
| 18 | 800,000 / 2 | — | 70.00 | — | 23.90 | — | 0.273 | — |
| 19 | 800,000 / 2 | 5,000,000 / 8 | 2,720.00 | 1,061.00 | 236.00 | 120.00 | 12.500 | 4.873 |
| 20 | 800,000 / 5 | 100,000 / 15 | 451.00 | 188.87 | 90.40 | 73.76 | 1.870 | 1.674 |

(*) In examples 15-20, the substance used is hylan, and not hyaluronate, as sodium hylan

The following can be concluded from the experimental data in Table I:

(1) All measured (actual) rheological parameters for sodium hyaluronate or hylan-poly(ethylene oxide) combinations are higher than the additive values calculated from the data for solutions of each of the components separately.

(2) Shear and dynamic viscosities are more sensitive parameters than are storage modulus.

(3) The degree of interaction between the two polymers does not depend only on the molecular weight of sodium hyaluronate or hylan.

(4) The degree of interaction seems to decrease with a decrease in the molecular weight of the Polyox. Compare, e.g. Examples 16 and 20.

## Claims

1. A water-based viscoelastic composition comprising a mixture of a hyaluronate and a linear homopolymer of ethylene oxide in water, characterized in that said hyaluronate has a molecular weight of about 50,000 up to about 1,000,000 and said homopolymer has a molecular weight of from about $1 \times 10^5$ up to about $5 \times 10^6$.

2. A composition according to Claim 1, wherein the hyaluronate is an alkali metal or alkaline earth metal hyaluronate.

3. A composition according to Claim 2, wherein the hyaluronate is sodium hyaluronate.

4. A composition according to Claim 2 or Claim 3, wherein the hyaluronate is a pure hyaluronic acid salt or a mixture of hyaluronates with proteins and other naturally occurring substances.

5. A composition according to any preceding claim, wherein the concentration of each component of the mixture is from about 0.01% to about 2% and the ratio by weight of the hyaluronate to the poly(ethylene oxide) is about 1:50 to 100:1.

6. A cosmetic formulation comprising from about 0.1 to about 50% by weight of a composition according to any preceding claim in combination with conventional cosmetic ingredients.

7. A cosmetic formulation according to Claim 6 which is a shaving gel, moisturizing cream, moisturizing hand lotion, or emollient moisturizing cream, or an eye drop formulation.

8. An eye drop formulation according to Claim 7, and further comprising an effective amount of at least one preservative.

9. A single dose of the eye drop formulation according to Claim 8, comprising about 0.1 - 0.5 ml of said formulation in a sealed, sterile container therefor.

10. A water-based viscoelastic composition comprising a mixture of a hylan and a linear homopolymer of ethylene oxide in water.

11. A composition according to Claim 10, wherein the hylan is an alkali metal or alkaline earth metal hylan.

12. A composition according to Claim 11, wherein the hylan is sodium hylan.

13. A composition according to any one of Claims 10-12, wherein the hylan has a molecular weight ranging from about $5 \times 10^5$ to about $6 \times 10^6$.

14. A cosmetic formulation comprising from about 0.1 to about 50% by weight of a composition according to any one of Claims 10-13 in combination with conventional cosmetic ingredients.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 138 572 (FIDIA SpA) * Page 3, lines 14-16; page 44, claim 6; page 21, examples 7-8 * | 1-3 | A 61 K 7/48 A 61 K 47/00 A 61 K 9/06 C 08 L 5/08 // (C 08 L 5/08 C 08 L 71:00 ) |
| Y | | 4-9 | |
| Y | US-A-4 582 865 (E. BALAZS) * Column 10, line 48 - column 11, line 10; claims 1,3 * | 1-14 | |
| D,Y | GB-A-2 160 097 (BIOMATRIX INC.) * Claims 1-17 * | 1-9 | |
| D,A | FR-A-2 478 468 (E. BALAZS) * Page 1, line 38 - page 2, line 3 * | 1-9 | |
| D,Y | GB-A-2 172 295 (BIOMATRIX INC.) * Page 2, lines 10-27 * | 10-14 | |
| X | FR-A-2 094 109 (I. LUBOWE) * Claims 1,9 * | 1-3 | |

TECHNICAL FIELDS
SEARCHED (Int. Cl.4)

A 61 K
C 08 L

| | | |
|---|---|---|
| The present search report has been drawn up for all claims | | |
| Place of search THE HAGUE | Date of completion of the search 14-03-1988 | Examiner FOERSTER W.K. |

EPO FORM 1503 03.82 (P0401)